# EUROPÄISCHE PATENTSCHRIFT

(11) **EP 2 735 321 B1**
(45) Veröffentlichungstag und Bekanntmachung des Hinweises auf die Patenterteilung: **15.07.2015**
(21) Anmeldenummer: 13193697.3
(22) Anmeldetag: 20.11.2013
(51) Int. Cl.: A61M 1/16

(54) **Kartuschenhalterung für eine Dialysemaschine**
Cartridge holder for a dialysis machine
Support de cartouches pour une machine de dialyse

(30) Priorität: 26.11.2012 DE 102012111428
(43) Veröffentlichungstag der Anmeldung: 28.05.2014
(73) Patentinhaber: B. Braun Avitum AG, 34212 Melsungen (DE)
(72) Erfinder: Iske, Andreas, 34320 Söhrewald (DE)
(74) Vertreter: Winter, Brandl, Fürniss, Hübner, Röss, Kaiser, Polte - Partnerschaft mbB

(56) Entgegenhaltungen:
- WO-A1-97/02056
- WO-A1-99/37342
- WO-A2-2009/064984
- JP-A- 2003 199 818
- JP-A- 2006 325 668

## Beschreibung

Die Erfindung betrifft eine Kartuschenhalterung für eine Dialysemaschine gemäß dem Oberbegriff des Patentanspruchs 1, wobei die Dialysemaschine zur Herstellung einer Dialysierflüssigkeit u. a. durch Auflösen eines in einer Kartusche befindlichen Salzes erfolgen kann. Des Weiteren betrifft die Erfindung eine Dialysemaschine mit einer Kartuschenhalterung.

In Dialysemaschinen erfolgt eine Aufbereitung einer Dialysierflüssigkeit, die durch einen Dialysator geleitet wird, wodurch einem Blut eines Patienten Schadstoffe entzogen werden können.

Die Druckschrift EP 0 278 100 B1 offenbart eine Kartuschenhalterung für eine Dialysemaschine. In der Dialysemaschine wird eine ein pulverförmiges Bikarbonat enthaltene Kartusche von Wasser durchströmt, wodurch eine Dialysierflüssigkeit durch ein Lösen des Bikarbonats in dem Wasser sowie zu mischen weiterer Komponenten geschaffen wird. Die Kartusche ist hierbei zwischen zwei verschwenkbaren Haltearmen angeordnet. Der in Vertikalrichtung gesehen untere Haltearm ist um eine Schwenkachse aus seiner horizontalen Betriebsstellung um etwa 90° in eine Spülstellung verschwenkbar. In seiner horizontalen Betriebsstellung stützt er sich mit einer Stützfläche an einer Stützschulter der Kartuschenhalterung ab. Der obere Haltearm ist ebenfalls bei entfernter Kartusche von seiner horizontalen Betriebsstellung um 90° nach unten, um eine Schwenkachse, in die Spülstellung verschenkbar. In der Spülstellung ist ein jeweiliger Fluidanschluss eines Haltearms mit einem Fluidanschluss der Kartuschenhalterung fluidisch verbunden, wobei die Fluidanschlüsse der Kartuschenhalterung zur Spülung - um die Kartuschenhalterung zu desinfizieren - kurz geschlossen sein können. Zum Einsetzen und Entfernen der Kartusche befindet sich der untere Haltearm in seiner horizontalen Betriebsstellung, während der obere Haltearm aus seiner horizontalen Betriebsstellung nach oben in eine Einlegestellung verschwenkt ist. Zum Einsetzen in den Kartuschenhalter taucht die Kartusche mit einem Nippel in den Fluidanschluss des unteren Haltearms und wird in etwa vertikal verschwenkt ist. Zum Einsetzen in den Kartuschenhalter taucht die Kartusche mit einem Nippel in den Fluidanschluss des unteren Haltearms und wird in etwa vertikal ausgerichtet. Im Anschluss daran wird der obere Haltearm in seine horizontale Betriebsstellung zurückverschwenkt, wodurch ein weiterer Nippel der Kartusche in den Fluidanschluss des oberen Haltearms eintaucht. Zum Entfernen der Kartusche wird der obere Haltearm von seiner horizontalen Betriebsstellung in eine Einlegestellung weggeschwenkt und die Kartusche vom unteren Haltearm entfernt, wobei es in der Praxis nachteilig notwendig ist, dass der untere Haltearm hierbei von einer Bedienperson gehaltert wird, da er sich sonst zusammen mit der Kartusche, insbesondere aufgrund einer reibschlüssigen Verbindung zwischen dem Nippel und dem Fluidanschluss, mitbewegen würde.

Die Druckschrift JP 2006-325668 offenbart einen Kartuschenhalter, der einen oberen schwenkbaren Arm und unten einen feststehenden Haltearm für eine Kartusche aufweist. Der obere, schwenkbare Arm kann über einen Knopf arretiert oder gelöst werden.

Die Druckschrift WO 2009/064984 offenbart eine Dialysemaschine mit einer Halteeinrichtung für Kartuschen mit zwei schwenkbaren Armen die nicht in verschiedenen Positionen verrastbar sind.

Die WO 99/37342 offenbart eine Sicherheitseinrichtung für eine Dialysemaschine.die eine gleichzeitige, unerwünschte Nutzung zweier Sorten Zusatzstoffe ausschließt. Es ist dabei eine Kartuschenhalterung mit zwei Haltearmen offenbart, die schwenkbar sind und zwar aus einer vertikalen in eine horizontale Position. Der Schwenkbereich ist bei 90°.

Damit die Bedienperson den unteren Haltearm halten kann, muss sie vom oberen Haltearm zum unteren Haltearm mit ihrer Hand umgreifen, da die andere Hand zum Haltern der Kartusche eingesetzt wird. Eine Einhandbedienung ist hierdurch nachteilig nicht möglich. Da der obere Haltearm beliebig um seine Schwenkachse verschwenkbar ist, kann dieser bei entfernter Kartusche, wenn er beispielsweise von dem Bedienpersonal losgelassen wird, unkontrolliert in seine Spülstellung schwenken, was bei einer hohen Schwenkgeschwindigkeit beispielsweise zu Beschädigungen an den Fluidanschlüssen oder an dem Haltearm führen kann. Des Weiteren ist nachteilig, dass die Haltearme nicht fest in Ihrer Spülposition gehaltert werden, was zu Fehlern bei der Desinfektion des Dialysegeräts führen kann. Zudem weisen die Haltearme keinen Überlastschutz auf, was bei einer Fehlbedienung der Dialysemaschine zu Beschädigungen führen kann. Zudem kann ein zu hoher Druck innerhalb der Kartusche, beispielsweise bedingt durch einen Systemfehler in der Dialysemaschine, zu einer Explosion der Kartusche führen, was wiederum zu einer Gefährdung von Patienten und Bedienpersonal, insbesondere im Bereich der Dialysemaschine, durch Lärm und Splitter führen kann.

Aus dem Stand der Technik sind des Weiteren Dialysemaschinen mit einem Kartuschenhalter bekannt, bei dem die Haltearme jeweils stirnseitig einen Haltevorsprung haben, die in der Spülstellung der Haltearme von einem an einem Gehäuse der Dialysemaschine ausgebildeten Halteelement hintergriffen sind, wodurch die Haltearme formschlüssig gehaltert sind. Nachteilig hierbei ist, dass die Haltearme hierdurch lediglich in der Spülstellung gehaltert werden.

Dem Gegenüber liegt der Erfindung die Aufgabe zu Grunde einen Kartuschenhalter und eine Dialysemaschine mit einem Kartuschenhalter zu schaffen, die die oben genannten Nachteile beseitigen.

Diese Aufgabe wird hinsichtlich des Kartuschenhalters gemäß den Merkmalen des Patentanspruchs 1 und hinsichtlich der Dialysemaschine gemäß den Merkmalen des Patentanspruchs 16 gelöst.

Sonstige vorteilhafte Weiterbildungen der Erfindung sind Gegenstand weiterer Unteransprüche.

Erfindungsgemäß hat ein Kartuschenhalter einer Dialysemaschine zwei jeweils um eine Schwenkachse verschwenkbare Haltearme zwischen denen eine Kartusche aufgenommen werden kann. Vorteilhafterweise wirkt zumindest ein Haltearm mit seiner Schwenkachse über eine Rastvorrichtung derart zusammen, dass er zumindest in einer Rastposition, insbesondere in einer Mehrzahl von Rastpositionen, gehaltert ist. Die Rastvorrichtung ist somit, insbesondere unmittelbar, zwischen dem Haltearm und der Schwenkachse vorgesehen.

Diese Lösung hat den Vorteil, dass die Rastvorrichtung äußerst kompakt ausgestaltet sein kann und dabei mehrere Rastpositionen zulassen kann. Im Unterschied dazu ist beim eingangs erläuterten Stand der Technik die Rastvorrichtung zwischen dem Haltearm und einem Gehäuse vorgesehen, wobei bei einem Verschwenken des Haltearms mit seinem Haltevorsprung dieser weg von dem Halteelement verschwenkt wird, wodurch hierbei nur eine Rastposition möglich ist.

In weiterer Ausgestaltung der Erfindung ist bei der Rastvorrichtung ein Rastelement an dem Haltearm und ein Rastelement an der Schwenkachse angeordnet. Die Rastelemente können hierbei über eine Kulissenführung in Wirkverbindung stehen. Dies hat den Vorteil, dass der Haltearm von einer Rastposition ausgelenkt werden kann und in eine nächste Rastposition verschwenkt werden kann, ohne dass beispielsweise ein Verriegelungselement mit einer Taste, einem Rasthaken oder einer Klemmung gelöst werden müssen. Ein derartiger Haltearm ist somit äußerst einfach zu Bedienen. Zum Verschwenken der Haltearme in ihre unterschiedlichen Rastpositionen ist somit durch die Kulissenführung eine Betätigung einer Endtriegelung obsolet. Des Weiteren ist bei der Kulissenführung vorteilhaft, dass der Haltearm, wenn auf ihn keine äußeren Kräfte wirken, in seiner Rastposition selbsttätig verbleibt. Die Rastvorrichtung führt zu einer logischen Wirkrichtung der einzelnen Bedienelemente bei einer Benutzung des Kartuschenhalters.

Als Rastpositionen für den Haltearm können eine Spülstellung, eine Einlegestellung und eine Betriebsstellung vorgesehen sein. Bei der Betriebsstellung sind Längsachsen der Haltearme im Wesentlichen im Parallelabstand zueinander und etwa in horizontaler Richtung ausgerichtet. Dagegen erstrecken sich die Längsachsen der Haltearme in der Spülstellung im Wesentlichen koaxial zueinander. In der Einlegestellung ist die Längsachse des in vertikaler Richtung gesehen unteren Haltearms etwa horizontal angeordnet und die Längsachse des oberen Haltearms ist zur Horizontalen angewinkelt und erstreckt sich vom unteren Haltearm weg. Für den in Vertikalrichtung gesehen oberen Haltearm können als Rastpositionen somit alle drei Stellungen vorgesehen sein und für den unteren Haltearm die Spülstellung und die Betriebsstellung. Durch die Rastvorrichtung kann die Kartusche äußerst einfach entnommen und eingelegt werden. Hierzu ist es lediglich notwendig, dass der obere Haltearm beispielsweise durch eine Hand einer Bedienperson in die Einlegestellung gebracht wird, wo er dann selbsttätig verbleibt, wodurch die Bedienperson mit ihrer Hand vom Haltearm auf die Kartusche umgreifen kann und diese entnehmen oder einlegen kann. Somit ist auf einfache Weise eine Einhandbedienung des Kartuschenhalters ermöglicht. Ferner verbleibt beim Entfernen der Kartusche aus dem Kartuschenhalter der untere Haltearm in seiner Betriebsstellung und schwenkt nicht, wie im eingangs erläuterten Stand der Technik, zusammen mit der Kartusche nach oben.

Bevorzugterweise ist die Rastvorrichtung in einem Innenraum des Haltearms angeordnet, wodurch sie vor äußeren Einflüssen geschützt ist. Des Weiteren kann die Rastvorrichtung hierdurch von Außen nicht einsehbar sein, was zu einer hohen ästhetischen Anmutung führt.

Vorzugsweise ist eines der Rastelemente der Rastvorrichtung mit einer Rastkraft beaufschlagt, wodurch das Rastelement mit seiner Kulissenbahn gegen eine Kulissenbahn des anderen Rastelements in Anlage haltbar ist. Somit werden auf einfache Weise die Rastelemente zueinander zwangsgeführt. Somit kann eines der Rastelemente festgelegt und das andere in Wirkrichtung der Rastkraft verschiebbar gelagert sein.

In weiterer Ausgestaltung der Erfindung sind die Kulissenbahnen der Kulissenführung derart ausgebildet, dass beim Beaufschlagen des Haltearms mit einem Drehmoment um seine Schwenkachse, beispielsweise durch die Bedienperson, das mit der Rastkraft beaufschlagte Rastelement ab einer bestimmten Größe des Drehmoments zum Wechseln der Rastpositionen entgegen einer Wirkrichtung der Rastkraft bewegt ist. Hierbei kann beispielsweise das zum Verstellen des Haltearms notwendige Drehmoment durch die Größe der Rastkraft eingestellt werden. Es ist beispielsweise denkbar, dass die Kulissenbahnen als eine Aneinanderreihung von schiefen Ebenen ausgebildet sind.

Mit Vorteil liegen in den Rastpositionen eine jeweilige Gleitfläche eines jeweiligen Rastelements aneinander, wodurch der Haltearm in der Rastposition gehaltert ist. Gleitflächen der Rastelemente sind somit derart angeordnet, dass der Haltearm in mehreren Rastpositionen festgelegt ist.

Vorzugsweise sind die Kulissenbahnen der Kulissenführung derart ausgebildet, dass der Haltearm durch die Rastkraft zumindest einer Rastposition selbsttätig bis zu einer bestimmten Auslenkung von dieser Rastposition zurückschwenkt. Vorzugsweise ist für jede Rastposition ein derartiger Tolleranzbereich vorgesehen. Das selbsttätige Zurückschwenken des Haltearms hat den Vorteil, dass beispielsweise eine Bedienperson den Haltearm nur grob in die gewünschte Rastposition verschwenken muss und der restliche Schwenkweg aufgrund der Rastkraft von dem Haltearm selbsttätig zurückgelegt wird. Des Weiteren ist hierdurch ein bestimmter Tolleranzbereich zugelassen, bei dem der Haltearm von seiner Rastposition aus verschwenkbar ist, ohne in die nächste Rastposition zu gelangen. Hierdurch können ferner Herstellerabhängige Längentolleranzen der Kartusche in der Betriebsstellung vorteilhafter Weise ausgeglichen werden. Außerdem werden durch das selbsttätige Zurückschwenken undefinierte Positionen der Haltearme, was zu einer Fehlfunktion der Dialysemaschine führen könnte, verhindert.

Die Rastkraft kann beispielsweise einfach durch eine sich am Haltearm oder an der Schwenkachse abstützenden, insbesondere einstellbaren Feder, insbesondere Spiralfeder, auf das Rastelement beaufschlagt werden.

Als bevorzugte Materialpaarungen für die Rastvorrichtung haben sich in der Praxis Rastelemente aus Edelstahl bewährt oder eine Materialpaarung bei der ein Rastelement aus Edelstahl und das andere Rastelement aus gut schmierendem Kunststoff, beispielsweise POM, besteht.

Es ist denkbar, dass die Kulissenführung der Rastvorrichtung derart ausgestaltet ist, dass der Haltearm nur innerhalb eines bestimmten Winkelbereichs verschwenkbar ist. Beispielsweise ist denkbar, dass der obere Haltearm von seiner Einlegeposition aus kaum mehr weiter weg von der Kartusche verschwenkbar ist, wodurch Fehlbedienungen des Kartuschenhalters vermieden werden.

Ferner ist denkbar, dass die Schwenkachsen innerhalb einer Halteschale befestigt sind und zusammen mit den Haltearmen eine vormontierte Einheit bilden. Die Haltearme und die Schwenkachsen sind durch die Halteschale dann zumindest abschnittsweise vor äußeren, unerwünschten mechanischen Einwirkungen geschützt. In der Halteschale kann für einen jeweiligen Haltearm ein Fluidanschluss vorgesehen sein, um den jeweiligen Haltearm mit seinem Fluidanschluss in die Spülstellung einzukoppeln. Ein jeweiliger Fluidanschluss des Haltearms kann beispielsweise mit einer Fluidleitung verbunden sein, die sich über die Schwenkachse in den Innenbereich des Haltearms bis zu seinem Fluidanschluss erstreckt.

Bevorzugterweise hat der Kartuschenhalter einen Überlastschutz für Fehlbedienungen bzw. bei einem Fehler im System. Dieser ist derart ausgestaltet, dass ein Druck innerhalb der Kartusche auf den in Vertikalrichtung gesehen oberen Haltearm wirkt und zu einer dem Druck äquivalenten Kraft führt, die wiederum ein Drehmoment auf den Haltearm ausübt. Übersteigt die Kraft bzw. das Drehmoment einen bestimmten Wert, so wird der Haltearm weg von der Betriebsstellung in Richtung seiner Einlegestellung bewegt, wodurch insbesondere die Kartusche druckentlastet wird.

Die Rastvorrichtung, insbesondere die Rastkraft, kann vorzugsweise auf die anliegenden Kräfte bzw. Drücke ausgelegt werden und somit zu geringen Bedienkräften führen.

In einer bevorzugten Ausführungsform ist eines der Rastelemente in einer Führung des Haltearms, insbesondere etwa in Richtung der Längsachse des Haltearms und insbesondere drehfest, geführt und über die Feder mit der Rastkraft beaufschlagt. Das andere Rastelement kann an einem in dem Innenraum des Haltearms angeordneten Achsenabschnitt der Schwenkachse ausgebildet sein. Das andere Rastelement ist entweder als zusätzliches Bauteil an der Schwenkachse oder einstückig mit der Schwenkachse ausgebildet. Durch diese Ausführungsform kann das gleitend geführte Rastelement somit etwa radial zur Schwenkachse auf das andere Rastelement wirken. Die Kulissenbahn des an dem Achsenabschnitt ausgebildeten Rastelements kann sich beispielsweise imn Umlaufrichtung um die Schwenkachse erstrecken, während die Kulissenbahn des gleitend im Haltearm geführten Rastelements auf einen zum Achsenabschnitt weisenden Endabschnitt des Rastelements ausgebildet sein kann.

In weiterer Ausgestaltung der Erfindung hat die Kulissenbahn des an dem Achsenabschnitt ausgebildeten Rastelements für jede Rastposition eine Gleitfläche, die jeweils derart ausgebildet ist, dass die Kulissenbahn des anderen Rastelements beim Verschwenken des Haltearms auf dieser abgleiten kann und hierdurch das andere Rastelement relativ zum Haltearm in oder gegen die Wirkrichtung der Rastkraft verschiebbar ist.

Alternativ ist denkbar, dass die Kulissenbahn des an dem Achsenabschnitt ausgebildeten Rastelements stirnseitig der Schwenkachse ausgebildet ist und die Kulissenbahn des gleitend im Haltearm geführten Rastelements an einer zum Achsenabschnitt weisenden Stirnseite des Rastelements vorgesehen ist. Die Rastelemente können somit etwa im Bereich der Schwenkachse angeordnet sein und sich parallel, insbesondere koaxial zueinander erstrecken.

In einer alternativen Ausführungsform ist eines der Rastelemente im Haltearm, insbesondere im Bereich der Schwenkachse, festgelegt und das andere Rastelement gleitend und drehfest auf der Schwenkachse geführt. Das andere Rastelement ist dann über die Feder mit der Rastkraft in Richtung des im Haltearm festgelegten Rastelements beaufschlagt.

Das auf der Schwenkachse geführte Rastelement kann vorrichtungstechnisch einfach ringförmig ausgestaltet sein und die Schwenkachse umgreifen. Zum drehfesten Führen des Rastelements kann in der Schwenkachse zumindest eine Längsnut eingebracht sein, in die das Rastelement mit einem sich radial nach Innen erstreckenden Vorsprung eingreifen kann.

Die Kulissenbahn des auf der Schwenkachse geführten Rastelements kann dann auf einer zum anderen Rastelement weisenden Ringstirnfläche ausgebildet sein, auf der die Kulissenbahn des anderen Rastelements anlegbar ist.

Erfindungsgemäß ist eine Dialysemaschine mit einem erfindungsgemäßen Kartuschenhalter vorgesehen.

Im Folgenden werden bevorzugte Ausführungsformen der Erfindung anhand von Zeichnungen näher erläutert. Es zeigen:
Fig. 1 in einer schematischen Seitenansicht einen erfindungsgemäßen Kartuschenhalter gemäß einer ersten Ausführungsform,
Fig. 2 in einer schematischen Draufsicht den Kartuschenhalter gemäß einer zweiten Ausführungsform ,
Fig. 3 in einem Längsschnitt den Kartuschenhalter gemäß einer dritten Ausführungsform,
Fig. 4 in einer perspektivischen Darstellung einen Ausschnitt des Kartuschenhalters aus Fig. 3,
Fig. 5 in einer perspektivischen Darstellung den Kartuschenhalter gemäß einer vierten Ausführungsform und
Fig. 6 in einer Draufsicht den Kartuschenhalter aus Fig. 5.

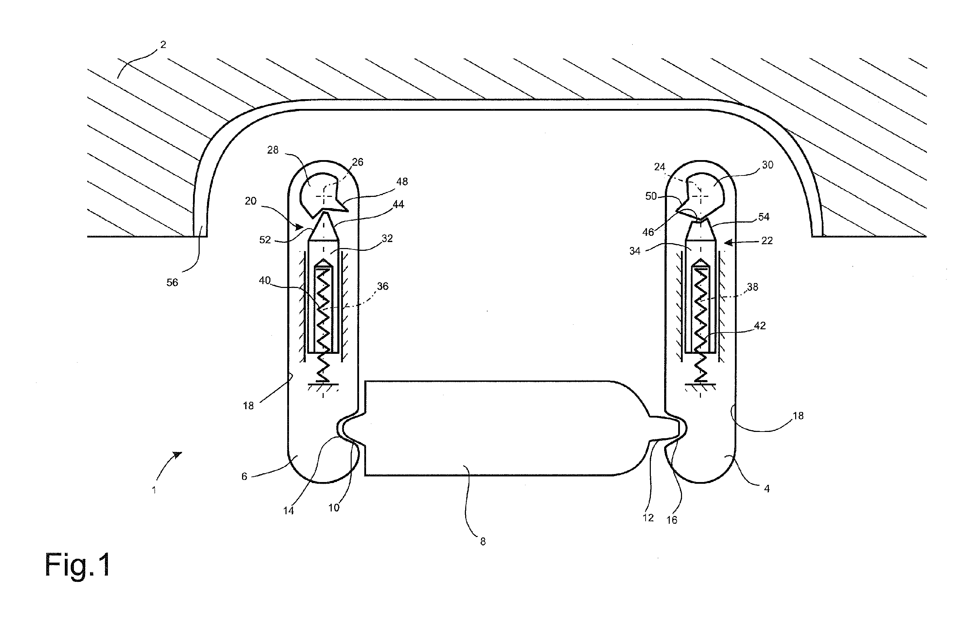

Gemäß Fig. 1 ist ein Kartuschenhalter 1 einer Dialysemaschine 2 stark vereinfacht dargestellt. Der Kartuschenhalter 1 hat zwei Haltearme 4 und 6, zwischen denen eine Kartusche 8 gehaltert ist. Diese kann beispielsweise ein pulvriges Bikarbonat-Konzentrat enthalten, das u. a. zur Bereitstellung einer Dialysierflüssigkeit benötigt wird. Die Kartusche 8 hat sowohl auf ihrer oberen als auch auf ihrer unteren Stirnseite etwa mittig jeweils einen Nippel 10 bzw. 12. Im eingesetzten Zustand der Kartusche 8 in den Kartuschenhalter 1 ist ein jeweiliger Nippel 10 und 12 jeweils in einen Fluidanschluss 14 bzw. 16 eines jeweiligen Haltearms 4 bzw. 6 eingetaucht. In einem jeweiligen Fluidanschluss 14 und 16 kann jeweils ein Dorn vorgesehen sein, der im eingesetzten Zustand der Kartusche 8 in einen jeweiligen Nippel 10 bzw. 12 der Kartusche 8 eindringt und hierdurch eine fluiddichte Verbindung zwischen der Kartusche 8 und den Fluidanschlüssen 14 und 16 schafft. Ein jeweiliger Fluidanschluss 14 und 16 ist dann jeweils mit einer nicht dargestellten Fluidleitung der Dialysemaschine 2 verbunden. Für weitergehende Informationen hinsichtlich der Kartusche 8 und den Fluidanschlüssen 14 und 16 wird beispielsweise auf die Druckschrift DE 198 52 982 C1 der Anmelderin verwiesen.

In einem Innenraum 18 eines jeweiligen Haltearms 4 und 6 ist erfindungsgemäß eine Rastvorrichtung 20 bzw. 22 vorgesehen. Über diese wirkt der jeweilige Haltearm 4 bzw. 6 derart mit seiner zugehörigen an der Dialysemaschine 2 fixierten Schwenkachse 24 bzw. 26 zusammen, dass er jeweils in eine Mehrzahl von Rastpositionen bringbar ist. Die Schwenkachsen 24 und 26 erstrecken sich etwa im Parallelabstand zueinander in einer gemeinsamen Schwenkachsenebene, die sich wiederum in etwa Vertikalrichtung erstreckt.

Eine jeweilige Rastvorrichtung 20 und 22 hat ein erstes fest mit der Schwenkachse 26 bzw. 24 verbundenes Rastelement 28 bzw. 30 und ein zweites gleitend im Haltearm 6 bzw. 8 geführtes Rastelement 32 bzw. 34. Die Rastelemente 32 und 34 sind kolben-/bolzenförmig ausgestaltet und in einer nicht dargestellten Führung (entspricht dem Innenraum 18) des Haltearms 6 bzw. 4 gleitend etwa in dessen Längsrichtung und etwa radial zur Schwenkachse 26 bzw. 24 geführt. Eine Längsachse der zweiten Rastelemente 32 und 34 erstreckt sich dabei etwa radial zur Schwenkachse 26 bzw. 24. Die bolzenförmigen zweiten Rastelemente 32 und 34 weisen jeweils eine von ihrer von der Schwenkachse 26 bzw. 24 abgewandten Stirnseite her eingebrachte Sacklochbohrung 36 bzw. 38 auf. In diese ist jeweils eine als Spiralfeder ausgebildete Feder 40 bzw. 42 eingesetzt, die sich jeweils innerhalb des Haltearms 6 bzw. 4 abstützen und das zweite Rastelement 32 bzw. 34 über eine Bodenfläche der jeweiligen Sacklochbohrung 36 bzw. 38 mit einer Rastkraft in Richtung der Schwenkachse 26 bzw. 24 beaufschlagen. Das zweite Rastelement 32 bzw. 34 wird hierdurch mit seiner zum ersten Rastelement 28 bzw. 30 weisenden Stirnseite 44 bzw. 66 in Anlage an das erste Rastelement 28 bzw. 30 gebracht.

Die Rastvorrichtungen 20 und 22 sind vorteilhafterweise als Kulissenführungen ausgestaltet. Hierzu hat das jeweils erste Rastelement 28 und 30 eine erste Kulissenbahn 48 bzw. 50 und die zweiten Rastelemente 32 und 34 jeweils eine zweite Kulissenbahn 52 bzw. 54. Die Kulissenbahnen 48 und 52 bzw. 50 und 54 liegen durch die Rastkraft der Feder 40 bzw. 42 aneinander an. Die ersten Kulissenbahnen 48 und 50 erstrecken sich hierbei am ersten Rastelement 28 bzw. 30 in Umlaufrichtung um die Schwenkachse 26 bzw. 24. Die zweiten Kulissenbahnen 52 und 54 der zweiten Rastelemente 32 bzw. 34 sind wiederum an der Stirnseite 44 bzw. 46 des zweiten Rastelements 32 bzw. 34 ausgebildet. Die Kulissenbahnen 48 und 52 der in Vertikalrichtung gesehen oberen Rastvorrichtung 20 unterscheiden sich in ihrer Form von den Kulissenbahnen 50 und 54 der unteren Rastvorrichtung 22, da die Haltearme 6 bzw. 4 eine unterschiedliche Anzahl von Rastpositionen aufweisen.

Als Rastpositionen für den in Vertikalrichtung gesehen oberen Haltearm 6 ist eine Spülstellung, eine Einlegestellung und eine Betriebsstellung vorgesehen und für den unteren Haltearm 4 eine Spülstellung und eine Betriebsstellung. Die Betriebsstellung der Haltearme 4 und 6 entspricht der Stellung gemäß Fig. 1. Die sich etwa radial von den Schwenkachsen 24 und 26 weg erstreckenden Längsachsen der Haltearme 4 und 6 erstrecken sich hierbei etwa in Parallelabstand zueinander und etwa in Horizontalrichtung. In der Spülstellung ist die Kartusche 8 entfernt und die Haltearme 4 und 6 sind von der in Fig. 1 gezeigten Betriebsstellung etwa um 90° hin zu einem Gehäuse 56 der Dialysemaschine 2 verschwenkt und weisen aufeinander zu. Ihre Längsachsen erstrecken sich dann etwa koaxial zueinander. In der Spülstellung sind die Spülanschlüsse 14 und 16 der Haltearme 6 bzw. 4 fluidisch mit im Gehäuse 56 angeordneten und in der Fig. 1 nicht dargestellten Fluidanschlüssen fluidisch verbunden. Hierdurch kann auf übliche Weise das Leitungssystem des Kartuschenhalters 1 desinfiziert und gespült werden. In der Einlegestellung ist der in Vertikalrichtung gesehen obere Haltearm 6 weg vom unteren Haltearm 4 bzw. von der Kartusche 8 geschwenkt. Der untere Haltearm 4 befindet sich hierbei in einer Stellung, die der Betriebsstellung entspricht. Durch das nach oben Wegschwenken des Haltearms 6 kann die Kartusche 8 in die Kartuschenhalterung 1 eingesetzt oder entfernt werden.

Zum Einsetzen oder Entfernen der Kartusche 8 greift eine Bedienperson mit ihrer Hand den Haltearm 6 und schwenkt diesen in die Einlegestellung. Hierbei gleitet das zweite Rastelement 32 mit seiner zweiten Kulissenbahn 52 entlang der ersten Kulissenbahn 48 des ersten Rastelements 28 derart, dass das zweite Rastelement 32 entgegen der Rastkraft der Feder 40 weg von der Schwenkachse 26 bewegt ist und bei Erreichen eines Totpunkts der ersten Kulissenbahn 48 sich wieder in Wirkrichtung der Rastkraft hin zur Schwenkachse 26 bewegt, bis die Einlegestellung erreicht ist. Somit muss auf den Haltearm 6 von der Bedienperson ein bestimmtes Drehmoment um die Schwenkachse 26 aufgebracht werden, um diesen von der Betriebsstellung zum Totpunkt zu verschwenken. Nach diesem Totpunkt kann das zweite Rastelement 32 durch die Rastkraft der Feder 40 selbsttätig in die Einlegestellung gleiten. Würde die Bedienperson den Haltearm 6 vor Erreichen des Totpunkts zwischen der Betriebsstellung und der Einlegestellung loslassen, so würde der Haltearm 6 aufgrund der Rastkraft der Feder 40 wieder zurück in die Betriebsstellung verschwenkt werden.

Befindet sich der Haltearm 6 in der Einlegestellung, so kann die Bedienperson mit ihrer Hand umgreifen und die Kartusche 8 in den Kartuschenhalter 1 einsetzen oder von diesem entfernen. Somit ist eine Einhandbedienung möglich. Soll der obere Haltearm 6 nach dem Entfernen der Kartusche 8 in seine Spülstellung geschwenkt werden, so wird dieser ausgehend von der Einlegestellung über die Betriebsstellung zur Spülstellung verschwenkt. Zwischen der Betriebsstellung und der Spülstellung ist ebenfalls ein Totpunkt vorgesehen, der über ein auf den Haltearm 6 und die Schwenkachse 26 wirkendes Drehmoment, das beispielsweise die Bedienperson aufbringt, überwunden werden muss. Wird der Haltearm 6 dann beispielsweise vor Erreichen des Totpunkts ausgehend von der Betriebsstellung von der Bedienperson losgelassen, so schwenkt er ebenfalls wieder selbsttätig durch die Rastkraft der Feder 40 in die Betriebsstellung zurück. Nach Überschreiten des Totpunkts kann er dann ohne Unterstützung eines Drehmoments ebenfalls selbsttätig durch die Rastkraft der Feder 40 in die Spülstellung schwenken. Die untere Rastvorrichtung 22 des Haltearms 4 ist ebenfalls derart ausgestaltet, dass der Haltearm 4 bei einer bestimmten Auslenkung von der Betriebsstellung in Richtung der Spülstellung vor Erreichen eines Totpunkts wieder in die Betriebsstellung durch die Rastkraft der Feder 42 zurück schwenkt, wenn auf ihn kein äußeres Drehmoment oder ein zu geringes Drehmoment wirkt. Das gleiche gilt, dass er zwischen dem Totpunkt und der Spülstellung ebenfalls selbsttätig durch die Rastkraft in die Spülstellung verschwenkt wird.

Somit sind die Kulissenbahnen 48, 52 bzw. 50, 54 derart ausgebildet, dass die Haltearme 6 bzw. 4 durch die Rastkraft zur jeweiligen Rastposition selbsttätig zurückschwenken, wenn sie nur höchstens bis zum Totpunkt ausgehend von dieser Rastposition ausgelenkt werden.

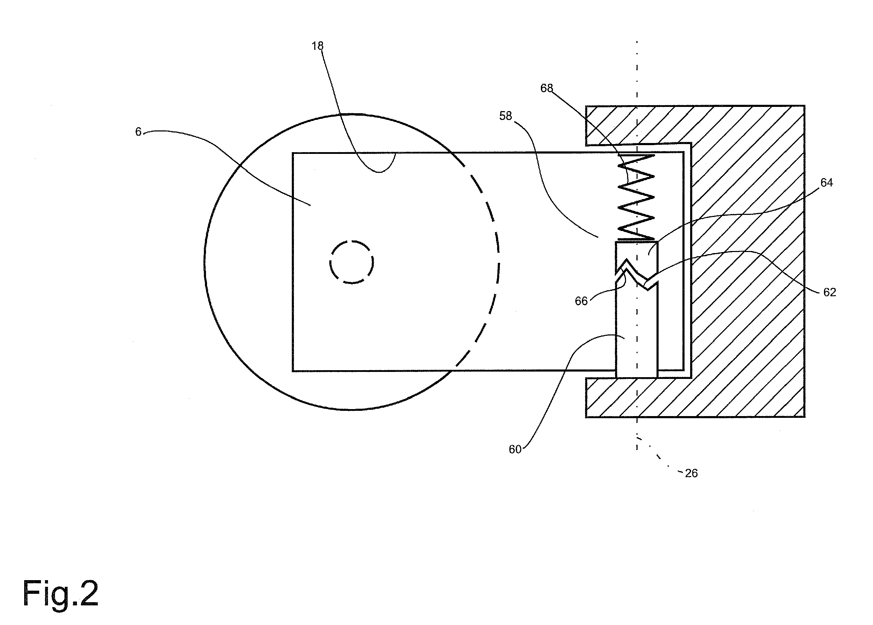

Gemäß Fig. 2 ist eine weitere Ausführungsform einer Rastvorrichtung 58 gezeigt. Diese wird anhand des oberen Haltearms 6 erläutert. Die Rastvorrichtung 58 erstreckt sich in der Schwenkachse 26. Das erste Rastelement 60 ist hierbei einstückig an einem in den Innenraum 18 des Haltearms 6 einkragenden Abschnitt der Schwenkachse 26 ausgebildet, wobei sich der Abschnitt etwa bis zur Mitte des Haltearms 6, in dessen Querrichtung betrachtet, erstreckt. Das erste Rastelement 60 hat hierbei eine erste Kulissenbahn 62, die stirnseitig der Schwenkachse 26 ausgebildet ist. Gegenüberliegend von der ersten Kulissenbahn 62 ist das zweite Rastelement 64 vorgesehen. Deren zweite Kulissenbahn 66 ist an deren Stirnseite gegenüberliegend von der ersten Kulissenbahn 62 angeordnet und liegt an dieser an. Der besseren Darstellbarkeit halber sind die Kulissenbahnen 62 und 66 gemäß Fig. 2 beabstandet zueinander dargestellt. Über eine von dem ersten Rastelement 60 weg weisenden Stirnseite ist das zweite Rastelement 64 mit der Rastkraft der Feder 68 beaufschlagt, die sich ebenfalls etwa koaxial zur Schwenkachse 26 erstreckt und sich am Haltearm 6 abstützt.

Beim Verschwenken des Haltearms 6 rotiert das zweite Rastelement 64 um die Schwenkachse 26 zusammen mit der Feder 68, wodurch die Kulissenbahnen 62 und 66 aufeinander abgleiten, da das erste Rastelement 60 fest mit der Schwenkachse 26 verbunden ist. Hierdurch kann der Haltearm 6 entsprechend der ersten Ausführungsform 1 in die unterschiedlichen Rastpositionen verschwenkt werden.

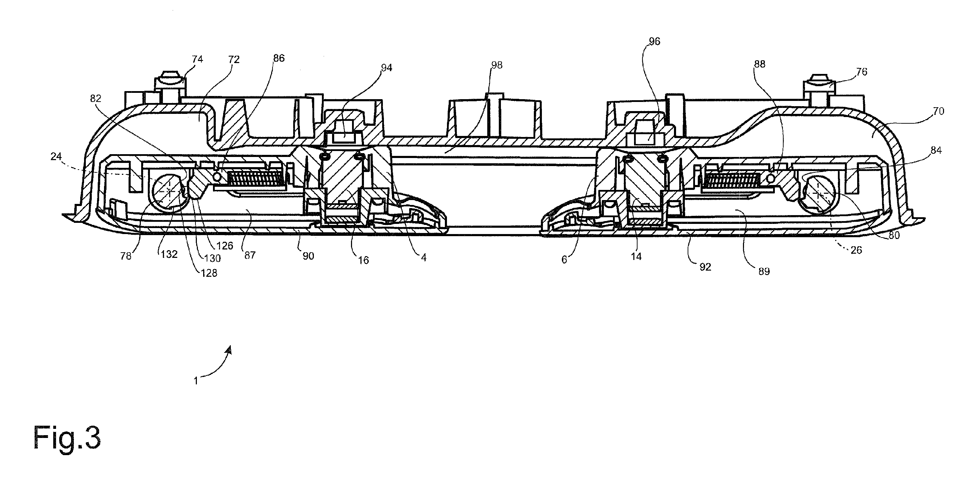

Gemäß der dritten Ausführungsform in Figur 3 hat der Kartuschenhalter 1 (wie er anhand der Fig. 1 beschrieben wurde) eine Halteschale 70, in der die Schwenkachsen 24 bzw. 26 zusammen mit den Hebeln 4 bzw. 6 befestigt sind. Die Halteschale 70 kann damit zusammen mit den Hebeln 4 und 6 eine vormontierbare Einheit bilden. In der gezeigten Spülstellung der Haltearme 4 und 6 sind diese im Wesentlichen vollständig in den konkaven Schalenraum 72 der Halteschale 70 aufgenommen, wodurch sie bei der Montage des Kartuschenhalters 1 in die Dialysenmaschine 2 geschützt sind. Selbstverständlich sind sie im eingetauchten Zustand auch im Einsatz der Dialysemaschine 2 vor äußeren mechanischen Einflüssen weitestgehend geschützt und können beispielsweise auch bei Nichtgebrauch der Dialysemaschine 2 in diese Position verschwenkt werden.

Die Schwenkachsen 24 und 26 sind jeweils an einem eine Rückseite der Halteschale 70 umgreifenden Bügel 74 bzw. 76 befestigt, die wiederum an der Halteschale 70 festgelegt sind.

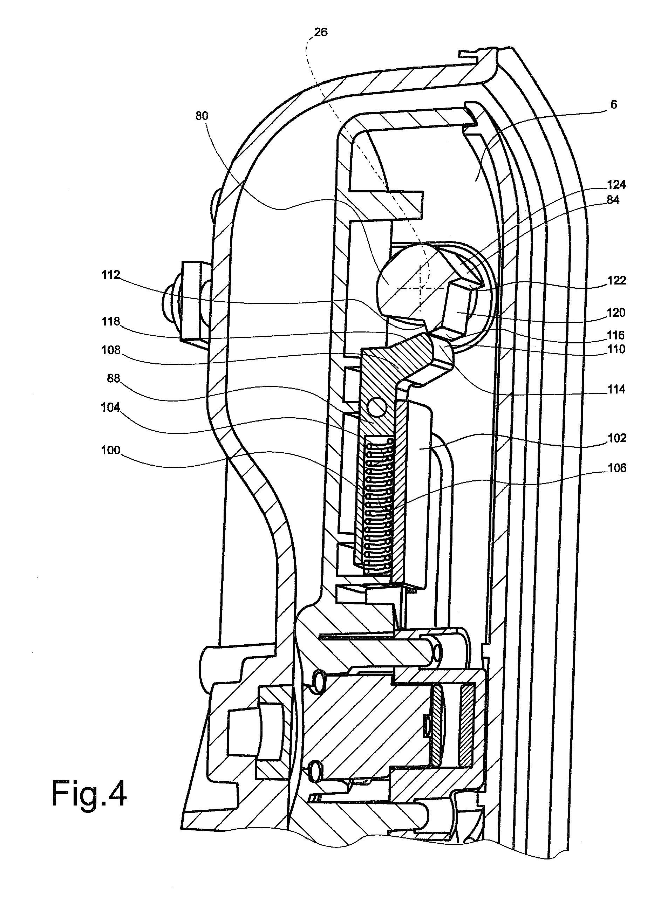

An der unteren und oberen Schwenkachse 24 und 26 sind entsprechend der ersten Ausführungsform aus Figur 1 die ersten Rastelemente 78 bzw. 80 vorgesehen, die jeweils eine um die Schwenkachse 24 bzw. 26 umlaufende erste Kulissenbahn 82 bzw. 84 aufweisen. Als zweites Rastelement 86 und 88 sind L-förmige Führungsstifte vorgesehen, die unten stehend in der Figur 4 näher erläutert sind. Ein jeweiliger Haltearm 4 und 6 hat ein schalenförmiges Gehäuse 87 bzw. 89, das von einem Deckel 90 bzw. 92 verschlossen ist.

Die Fluidanschlüsse 14 und 16 sind schematisch in der Figur 3 dargestellt und jeweils im vom ersten Rastelement bzw. von der Schwenkachse 80 bzw. 78 wegweisenden Endabschnitt des Haltearms 6 bzw. 4 angeordnet. In der gezeigten Spülstellung der Haltearme 4 und 6 sind die Fluidanschlüsse 16 bzw. 14 mit Fluidanschlüssen 94 bzw. 96 fluidisch verbunden. Die Fluidanschlüsse 94 und 96 sind in der Figur 3 nur schematisch angedeutet. Sie sind hierbei in einem Gehäuseboden 98 der Halteschale 70 ausgebildet. In der Spülstellung liegen die Haltearme 4 und 6 im Bereich ihrer Fluidanschlüsse 16 bzw. 14 am Gehäuseboden 98 an.

Anhand der Figur 4 wird die Rastvorrichtung gemäß der Ausführungsform des Kartuschenhalters 1 aus Figur 2 oder 3 näher erläutert. Das stiftförmige zweite Rastelement 88 des oberen Haltearms 6 ist etwa L-förmig ausgestaltet. Es hat hierbei einen ersten Schenkel 100, der gleitend in Längsrichtung des Haltearms 6 in einer in diesem ausgebildeten Führung 102 geführt ist. Das zweite Rastelement 88 hat hierbei eine Aussparung 104, in die die als Spiralfeder ausgebildete Feder 106 eingesetzt ist. Die Feder 106 stützt sich dabei am Haltearm 6 ab und beaufschlagt das zweite Rastelement 88 über einen Boden der Aussparung 104 mit der Rastkraft hin in Richtung zur Schwenkachse 26. Der Schenkel 100 ist etwas versetzt zur Schwenkachse 26 angeordnet. Damit das zweite Rastelement 88 an der ersten Kulissenbahn 84 des ersten Rastelements 80 anliegen kann, ist ein zweiter Schenkel 108 vorgesehen, der sich ausgehend von dem Schenkel 100 hin zum ersten Rastelement 80 erstreckt. Stirnseitig des zweiten Schenkels 108 ist die zweite Kulissenbahn 110 des zweiten Rastelements 88 ausgebildet.

Im Folgenden werden die unterschiedlichen Gleitflächen der ersten Kulissenbahn 84 des ersten Rastelements 80 und der zweiten Kulissenbahn 110 des zweiten Rastelements 88 näher erläutert. Die erste Kulissenbahn 84 hat für die Spülstellung des Hebels 6 eine sich etwa in Radialrichtung zur Schwenkachse 26 erstreckende erste Gleitfläche 112. Diese ist dabei beabstandet zu einer von den Schwenkachsen 24 und 26 aus Figur 3 aufgespannten Schwenkachsenebene angeordnet und zwar auf einer Seite der Schwenkachsenebene, die vom Gehäuseboden 98 aus Figur 3 weg weist. Die erste Gleitfläche ist hierbei etwa rechteckförmig und eben ausgestaltet, wobei sich zwei Seiten der Gleitfläche etwa parallel zur Schwenkachse 94 und zwei Seiteninsbesondere Schmalseiten - etwa radial zur Schwenkachse 26 erstrecken. In der gezeigten Spülstellung des Hebels 6 wirkt eine erste Gleitfläche 114 der zweiten Kulissenbahn 110 des zweiten Rastelements 88 mit der ersten Gleitfläche 112 der ersten Kulissenbahn 84 zusammen, wobei die Gleitflächen 112, 114 im Wesentlichen aneinander anliegen, was in den Figuren 3 und 4 der besseren Darstellbarkeit halber nur angedeutet ist. Die erste Gleitfläche 114 des zweiten Rastelements 88 erstreckt sich dann in der gezeigten Spülstellung etwa parallel zur ersten Gleitfläche 112 des ersten Rastelements 80 und weist eine ähnliche Größe auf.

In Schwenkrichtung gesehen ausgehend von der Spülstellung hin zur Betriebsstellung schließt sich an die erste Gleitfläche 112 der ersten Kulissenbahn 84 eine Übergangsfläche 116 an. Diese ist etwa 90° zur ersten Gleitfläche 112 angeordnet und weist in Umfangrichtung der Schwenkachse 26 gesehen etwa eine gleiche Breite wie die erste Gleitfläche 112 auf. Über diese gleitet der Schenkel 108 des zweiten Rastelements 26, wenn der Haltearm 6 von der Spülstellung in Richtung Betriebsstellung und umgekehrt verschwenkt wird. Der Schenkel 108 gleitet hierbei mit einer sich etwa im Parallelabstand zur Schwenkachse 26 erstreckenden Kante ab, die zwischen seiner ersten Gleitfläche 114 und seiner zweiten etwa in 90° zur ersten Gleitfläche 114 ausgebildeten Gleitfläche 118 vorgesehen ist. Die zweite Gleitfläche 118 erstreckt sich dann etwa ausgehend von der ersten Gleitfläche 114 hin zum anderen Schenkel 100 des zweiten Rastelements 88 und weist dabei etwa in Richtung des ersten Rastelements 80.

Nach der Übergangsfläche 116 schließt sich in Schwenkrichtung von der Spülstellung hin zur Betriebsstellung eine zweite Gleitfläche 120 an. In die gelangt das zweite Rastelement 88 mit seiner zweiten Gleitfläche 118 in Anlage, wenn es mit seiner Kante die Übergangsfläche 116 überwunden hat. Die zweite Gleitfläche 120 ist etwa in Umfangsrichtung gesehen viermal so lang ausgebildet wie die erste Gleitfläche 112. Ein Abstand der zweiten Gleitfläche 120 von der von den Schwenkachsen 24 und 26 aufgespannten Schwenkachsenebene wird hierbei mit Abstand zur Übergangsfläche 116 größer, wodurch die zweite Gleitfläche 120 etwas zur Schwenkebene angestellt ist. In der Betriebsstellung liegen die zweiten Gleitflächen 118 und 120 im Wesentlichen aneinander an.

In Schwenkrichtung von der Betriebsstellung zur Einlegestellung des Haltearms 6 schließt sich an die zweite Gleitfläche 120 des ersten Rastelements 80 eine weitere Übergangsfläche 122 an. Diese weist etwa die gleiche Fläche wie die erste Übergangsfläche 116 auf und erstreckt sich ausgehend von der zweiten Gleitfläche 120 etwa in Radialrichtung weg von der Schwenkachse 26.

An diese Übergangsfläche 122 schließt sich dann eine dritte Gleitfläche 124 an. Deren Fläche entspricht etwa der Fläche der zweiten Gleitfläche. In der Einlegestellung liegt dann das zweite Rastelement 88 mit seiner zweiten Gleitfläche 118 im Wesentlichen an die dritte Gleitfläche 124 an. Ein Abstand der dritten Gleitfläche 124 zur Schwenkebene verringert sich etwa linear ausgehend von der Übergangsfläche 122.

Die erste Kulissenbahn 84 des ersten Rastelements 80 weist somit eine zahnartige Struktur auf, wobei sich die Zähne radial von der Schwenkachse 26 wegerstrecken. Die erste Gleitfläche 112, die Übergangsfläche 116 und die zweite Gleitfläche 120 bilden hierbei einen ersten Zahn und die Übergangsfläche 122 und die dritte Gleitfläche 124 einen zweiten Zahn. Somit werden die vorderen und hinteren Zahnflanken der Zähne zur Positionierung des beweglichen Rastelements 88 eingesetzt. Die Zahnflanken sind hierbei als schiefe Ebenen ausgestaltet, damit der Hebelarm 6 ab einem bestimmten Drehmoment von einem Zahn zum nächsten gleiten kann.

Dagegen weist das in der Figur 3 gezeigte untere erste Rastelement 78 nur einen Zahn auf. Das erste Rastelement 78 und das zweite Rastelement 86 des Haltearms 4 sind im Wesentlichen entsprechend denen des Haltearms 6 nachgebildet. Das zweite Rastelement 86 hat hier eine erste Gleitfläche 126 und eine zweite Gleitfläche 128, die etwa rechtwinklig zueinander angeordnet sind, wobei sich die zweite Gleitfläche 128 etwa quer zur Längsachse des Haltearms 4 erstreckt. Das erste Rastelement 78 hat ebenfalls eine erste und zweite Gleitfläche 130 und 132, die sich beide etwa parallel zur Schwenkachsenebene erstrecken. Die ersten Gleitflächen 126 und 130 des zweiten und ersten Rastelements 86 bzw. 78 liegen hierbei in der Spülstellung etwa aneinander an, während die zweiten Gleitflächen 128 und 132 in der Betriebsstellung im Wesentlichen aneinander anliegen. Zwischen den Gleitflächen 130 und 132 des ersten Rastelements 78 ist eine Übergangsfläche vorgesehen.

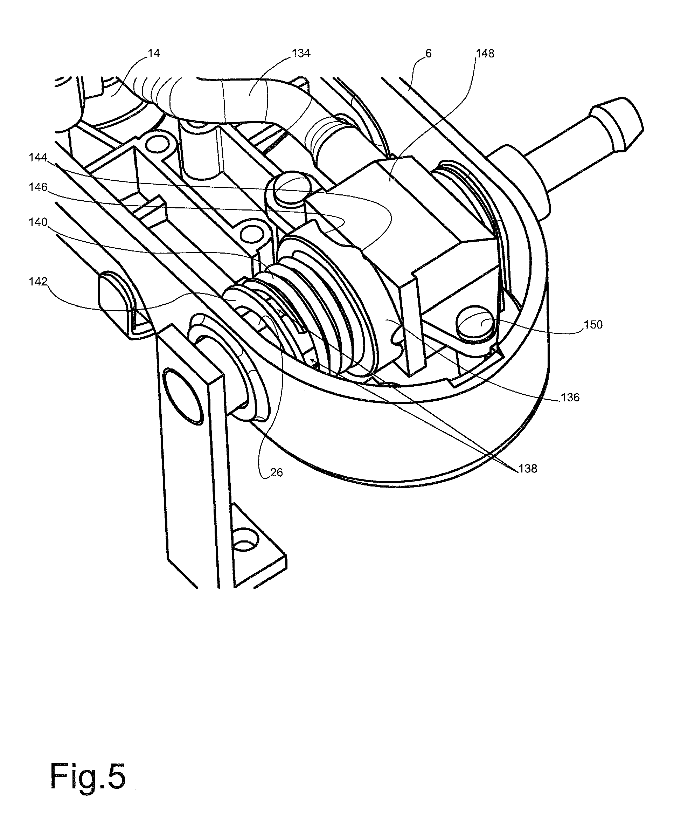

Gemäß Figur 5 ist der obere Haltearm 6 dargestellt. Hierbei ist die Fluidleitung 134 für den Fluidanschluss 14 erkennbar. Diese erstreckt sich innerhalb des Haltearms 6 und ist etwa koaxial zur Schwenkachse 28 aus dem Haltearm 6 herausgeführt.

Das erste Rastelement 136 des Haltearms 6 ist in Axialrichtung gleitend auf der Schwenkachse 26 geführt und ringförmig ausgebildet. Das Rastelement 136 ist hierbei drehfest mit der Schwenkachse 26 verbunden, indem in diese eine Mehrzahl von auf einem Teilkreis angeordneter Längsnuten 138 ausgebildet sind, in die das erste Rastelement 136 mit sich radial nach Innen erstreckenden Vorsprüngen bzw. Radialvorsprüngen eingreift. Die Schwenkachse 26 ist von der als Spiralfeder ausgebildeten Feder 140 umfasst, die das Rastelement 136 mit einer Rastkraft beaufschlagt und sich an einem an der Schwenkachse 26 festgelegten Federteller 142 abstützt. Auf einer von der Feder 140 abgewandten Ringstirnfläche 144 des ersten Rastelements 136 ist die erste Kulissenbahn 146 ausgebildet. Gegenüberliegend von der Ringstirnfläche 144 ist das zweite Rastelement 148 vorgesehen, das über eine Schraubverbindung 150 fest mit dem Haltearm 6 verschraubt ist.

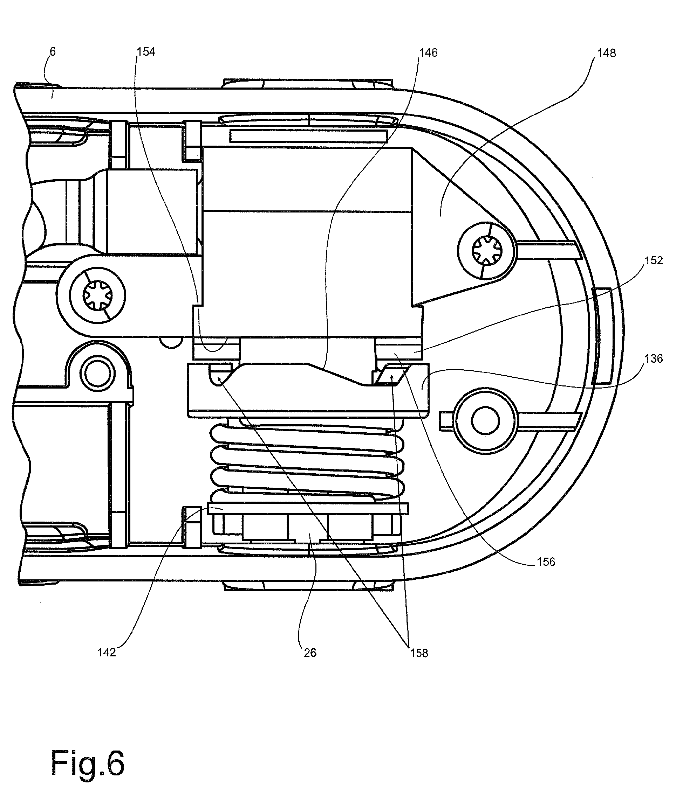

Gemäß Figur 6 ist der besseren Darstellbarkeit halber das erste Rastelement 136 vom zweiten Rastelement 148 beabstandet dargestellt. Die zweite Kulissenbahn 152 ist an dem zweiten Rastelement 148 an einer sich quer zur Schwenkachse 26 erstreckenden und zum ersten Rastelement 136 weisenden Stirnfläche 154 ausgebildet. Die Kulissenbahn 152 ist durch einen Steg 156 gebildet, der sich etwa in Längsrichtung des Haltearms 6 erstreckt und die Schwenkachse 26 schneidet. Der Steg 156 und somit die zweite Kulissenbahn 152 taucht dann in in der ersten Kulissenbahn 146 ausgebildete Nuten 158 ein.

Der Federteller 142 ist in Axialrichtung der Schwenkachse 26 verschiebbar und in einer gewünschten Position festlegbar, wodurch die Rastkraft der Feder 140 einstellbar ist.

Offenbart ist, insbesondere erfindungsgemäß, ein Kartuschenhalter einer Dialysemaschine. Diese hat jeweils zwei um eine Schwenkachse verschwenkbare Haltearme zum Haltern einer Kartusche. Die Haltearme sind jeweils über eine Rastvorrichtung mit ihrer Schwenkachse gekoppelt. Durch die Rastvorrichtung ist ein jeweiliger Haltearm zumindest in eine Rastposition bringbar.

### Bezugszeichenliste

- 1: Kartuschenhalter
- 2: Dialysemaschine
- 4, 6: unterer / oberer Haltearm
- 8: Kartusche
- 10, 12: Nippel
- 14, 16: Fluidanschluss
- 18: Innenraum
- 20, 22: Rastvorrichtung
- 24, 26: Schwenkachse
- 28, 30: erstes Rastelement / Vor- oder Rücksprünge an der Schwenkachse 24, 26
- 32, 34: zweites Rastelement / Bolzen / Kolben
- 36, 38: Sacklochbohrung / Längsloch
- 40, 42: Vorspannfeder
- 44, 46: Rastabschnitt aufweisende Stirnseite
- 48, 50: erste Kulissenbahn / Vor-/Rücksprünge
- 52, 54: zweite Kulissenbahn / Rastabschnitt
- 56: Gehäuse
- 58: Rastvorrichtung
- 60: erstes Rastelement
- 62: erste Kulissenbahn
- 64: zweites Rastelement
- 66: zweite Kulissenbahn
- 68: Vorspannfeder
- 70: Halteschale
- 72: Schalenraum
- 74, 76: Befestigungsbügel
- 78, 80: erstes Rastelement
- 82, 84: erste Kulissenbahn
- 86, 88: zweites Rastelement
- 87, 89: schalenförmiges Gehäuse
- 90, 92: Deckel
- 94, 96: Fluidanschluss
- 98: Gehäuseboden
- 100: erster Schenkel
- 102: Führung
- 104: Aussparung
- 106: Feder
- 108: zweiter Schenkel
- 110: zweite Kulissenbahn
- 112: erste Gleitfläche
- 114: erste Gleitfläche
- 116: Übergangsfläche
- 118: zweite Gleitfläche
- 120: zweite Gleitfläche
- 122: Übergangsfläche
- 124: dritte Gleitfläche
- 126: erste Gleitfläche
- 128: zweite Gleitfläche
- 130: erste Gleitfläche
- 132: zweite Gleitfläche
- 134: Fluidleitung
- 136: erstes Rastelement
- 138: Längsnuten
- 140: Feder
- 142: Federteller
- 144: federabgewandte Ringstirnfläche
- 146: erste Kulissenbahn
- 148: zweites Rastelement
- 150: Schraubverbindung
- 152: zweite Kulissenbahn
- 154: Stirnfläche
- 156: Steg
- 158: Nuten

## Patentansprüche

1. Kartuschenhalter einer Dialysemaschine mit zwei um jeweils eine Schwenkachse (24, 26) verschwenkbaren Haltearmen (4, 6), die beide in Höhenrichtung schwenkbar sind, **dadurch gekennzeichnet, dass** beide Haltearme (4, 6) jeweils über einen Rastmechanismus mit der zugehörigen Schwenkachse zusammenwirken, der die Haltearme (4, 6) in einer Mehrzahl von Rastpositionen in beide Schwenkrichtungen festhält, wobei zumindest ein Haltearm (4) in wenigstens drei höhenbeabstandete Positionen verrastbar ist, nämlich einer Einlegeposition, einer Betriebsposition und einer Spülposition, die durch Verschwenken des Haltearms (4) um die horizontale Schwenkachse mit fester Ausrichtung erreichbar sind.

2. Kartuschenhalter nach Anspruch 1, wobei bei der Rastvorrichtung (20, 22) ein Rastelement (32, 34) an dem Haltearm (6, 4) und ein Rastelement (28, 30) an der Schwenkachse (24, 26) vorgesehen ist.

3. Kartuschenhalter nach Anspruch 2, wobei die Rastelemente (28, 32; 30, 34) über eine Kulissenführung (48, 52; 50, 54) in Wirkverbindung stehen.

4. Kartuschenhalter nach einem der Ansprüche 1 bis 3, wobei als Rastpositionen eine Spülstellung, eine Einlegestellung und eine Betriebsstellung vorgesehen sind.

5. Kartuschenhalter nach einem der vorhergehenden Ansprüche, wobei die Rastvorrichtung (20, 22) in einem Innenraum (18) des Haltearms (4, 6) angeordnet ist.

6. Kartuschenhalter nach einem der Ansprüche 2 bis 5, wobei eines der Rastelemente (32, 34) der Rastvorrichtung (20, 22) mit einer Rastkraft beaufschlagt ist, durch die das Rastelement (32, 34) mit seiner Kulissenbahn (52, 54) gegen eine Kulissenbahn (48, 50) des anderen Rastelements (28, 30) in Anlage gehalten ist.

7. Kartuschenhalter nach Anspruch 6, wobei die Kulissenbahnen (48, 52; 50, 54) der Kulissenführung derart ausgebildet sind, dass beim Beaufschlagen des Haltearms (60.4) mit einem Drehmoment um seine Schwenkachse (26, 24) das mit der Rastkraft beaufschlagte Rastelement (32, 34) ab einer bestimmten Größe des Drehmoments zum Wechseln der Rastpositionen entgegen einer Wirkrichtung der Rastkraft bewegt ist.

8. Kartuschenhalter nach Anspruch 6 oder 7, wobei die Kulissenbahnen (48, 52; 50, 54) der Kulissenführung derart ausgebildet sind, dass der Haltearm (6, 4) durch die Rastkraft zumindest zu einer Rastposition selbsttätig bis zu einer bestimmten Auslenkung von dieser Rastposition zurückschwenkt.

9. Kartuschenhalter nach einem der Ansprüche 6 bis 8, wobei die Rastkraft durch eine sich am Haltearm (6, 4) oder an der Schwenkachse (26, 24) abstützenden Feder (40, 42; 140) auf das Rastelement (32, 34; 136) beaufschlagt ist.

10. Kartuschenhalter nach Anspruch 9, wobei eines der Rastelemente (32, 34) in einer Führung des Haltearms (6, 4) gleitend geführt ist und über die Feder (40, 42) mit der Rastkraft beaufschlagt ist, und wobei das andere Rastelement (28, 30) an einem in den Innenraum (18) des Haltearms (6, 4) angeordneten Achsenabschnitt (24, 26) der Schwenkachse (24, 26) ausgebildet ist.

11. Kartuschenhalter nach Anspruch 10, wobei sich die Kulissenbahn (48, 50) des an dem Achsenabschnitt ausgebildeten Rastelements (28, 30) sich in Umlaufrichtung um die Schwenkachse (26, 24) erstreckt und die Kulissenbahn (52, 54) des geleitend im Haltearm (6, 4) geführten Rastelements (32, 34) auf einen zum Achsenabschnitt weisenden Endabschnitt des Rastelements (32, 34) ausgebildet ist.

12. Kartuschenhalter nach Anspruch 10, wobei sich die Kulissenbahn (62) des an dem Achsenabschnitt ausgebildeten Rastelements (60) stirnseitig der Schwenkachse (26) erstreckt und die Kulissenbahn (66) des gleitend im Haltearm (6) geführten Rastelements (64) auf einer zum Achsenabschnitt weisenden Stirnseite des Rastelements (64) ausgebildet ist.

13. Kartuschenhalter nach Anspruch 9, wobei eines der Rastelemente (148) der Rastvorrichtung im Haltearm (6) festgelegt ist, wobei das andere Rastelement (136) gleitend und drehfest auf der Schwenkachse (26) geführt und über die Feder (140) mit der Rastkraft mit der Richtung des im Haltearm (6) festgelegten Rastelements (148) beaufschlagt ist.

14. Kartuschenhalter nach Anspruch 13, wobei das auf der Schwenkachse (36) geführte Rastelement (136) ringförmig ausgestaltet ist und die Schwenkachse (26) umkreist.

15. Kartuschenhalter nach Anspruch 1, wobei
zumindest eine Schwenkachse (24, 26) mit einer Anzahl von in Schwenkrichtung beabstandeten Vor- und/oder Rücksprüngen ausgebildet ist, welche die Rastpositionen definieren und
die Rastvorrichtung (20, 22) zumindest einen axial verschiebbar im zugehörigen Haltearm gelagerten Kolben (34) hat, der an seiner Stirnseite mit einem zu den Vor- und/oder Rücksprüngen kompatiblen Rastabschnitt ausgebildet ist und stirnseitig gegen die eine Schwenkachse (24, 26) federvorgespannt ist.

16. Dialysemaschine mit einem Kartuschenhalter (1) gemäß einem der vorhergehenden Ansprüche.

## Claims

1. Cartridge holder of a dialysis machine, with two holding arms (4, 6), each pivotable around a pivot axis (24, 26), both pivotable in a height direction, **characterised in that** both holding arms (4, 6) each cooperate with the associated pivot axis that holds the holding arms (4, 6) in a number of locking positions in both pivot directions via an locking mechanism, whereby at least one holding arm (4) can be locked in at least three height spaced positions, namely an insertion position, an operating position and a rinsing position, which can be reached by pivoting the holding arm (4) around the horizontal pivot axis with a fixed alignment.

2. Cartridge holder according to claim 1, whereby an locking element (32, 34) is envisaged on the holding arm (6, 4) and an locking element (28, 30) on the pivot axis (24, 26) for the locking device (20, 22).

3. Cartridge holder according to claim 2, whereby the locking elements (28, 32; 30, 34) stand in operative connection via a sliding block guide (48, 52; 50, 54).

4. Cartridge holder according to one of the claims 1 to 3, whereby a rinsing position, an insertion position and an operating position are envisaged as locking positions.

5. Cartridge holder according to one of the preceding claims, whereby the locking device (20, 22) is located in an interior space (18) of the holding arm (4, 6).

6. Cartridge holder according to one of the claims 2 to 5, whereby an locking force is applied to one of the locking elements (32, 34) of the locking device (20, 22), with which the locking element (32, 34) is held in abutment against a sliding block guide (48, 50) of the other locking element (28, 30) with its sliding block guide (52, 54).

7. Cartridge holder according to claim 6, whereby the sliding block guides (48, 52; 50, 54) of the sliding block are designed in such a way that when a torque is applied to the holding arm (60.4) around its pivot axis (26, 24), the locking element (32, 34) placed under the locking force is moved to change the locking position against an effective direction of the locking force from a specific size of said torque.

8. Cartridge holder according to claim 6 or 7, whereby the sliding block guides (48, 52; 50, 54) of the sliding block are designed in such a way that the holding arm (6, 4) will pivot back from this locking position by means of the locking force into at least one locking position, independently up to a specific displacement.

9. Cartridge holder according to one of the claims 6 to 8, whereby the locking force is applied to the locking element (32, 34; 136) by a spring (40, 42; 140) supported on a holding arm (6, 4) or on the pivot axis (26, 24).

10. Cartridge holder according to claim 9, whereby one of the locking elements (32, 34) is slidingly guided in the guide of the holding arm (6, 4) and the locking force applied via the spring (40, 42), and whereby the other locking element (28, 30) is formed on an axis section (24, 26) of the pivot axis (24, 26) located in the interior space (18) of the holding arm (6, 4).

11. Cartridge holder according to claim 10, whereby the sliding block guide (48, 50) of the locking element (28, 30) formed on the axis section extends in a circumferential direction around the pivot axis (26, 24), and the sliding block guide (52, 54) of the locking element (32, 34) slidingly held in the holding arm (6, 4) is formed on an end section of the locking element (32, 34) facing the axis section.

12. Cartridge holder according to claim 10, whereby the sliding block guide (62) of the locking element (60) formed on the axis section extends along the facing side of the pivot axis (26), and the sliding block guide (66) of the locking element (64) slidingly guided in the holding arm (6) is formed on a facing side of the locking element (64) facing the axis section.

13. Cartridge holder according to claim 9, whereby one of the locking elements (148) of the locking device is fixed in the holding arm (6), whereby the other locking element (136) is slidingly and non-rotatably guided on the pivot axis (26) and subjected to the locking force via the spring (140) in the direction of the locking element (148) fixed in the holding arm (6).

14. Cartridge holder according to claim 13, whereby the locking element (136) guided on the pivot axis (36) is of an annular design and circles the pivot axis (26).

15. Cartridge holder according to claim 1, whereby at least one pivot axis (24, 26) is formed with a number of projections and/or recesses spaced apart in pivot direction, which define the locking position, and
the locking device (20, 22) has at least one axially displaceable piston (34) mounted in the associated holding arm, formed with an locking section compatible with the projections and/or recesses at its facing side, and pre-tensioned by means of a spring against a pivot axis (24, 26) on its facing side.

16. Dialysis machine with a cartridge holder (1) according to one of the preceding claims.

## Revendications

1. Support de cartouche pour une machine de dialyse ayant deux bras de support (4, 6) pouvant pivoter respectivement autour d'un axe de pivotement (24, 26), qui peuvent tous les deux pivoter dans le sens de la hauteur, **caractérisé en ce que** les deux bras de support (4, 6) coopèrent chacun, par le biais d'un mécanisme de verrouillage, avec l'axe de pivotement y afférent, qui maintient les bras de support (4, 6) dans une pluralité de positions de verrouillage dans les deux sens de pivotement, au moins un bras de support (4) pouvant être verrouillé dans au moins trois positions écartées en hauteur, à savoir une position d'insertion, une position de fonctionnement et une position de rinçage, qui peuvent être atteintes en faisant pivoter le bras de support (4) autour de l'axe de pivotement horizontal ayant une orientation fixe.

2. Support de cartouche selon la revendication 1, dans lequel il est prévu, pour le dispositif de verrouillage (20, 22), un élément de verrouillage (32, 34) sur le bras de support (6, 4) et un élément de verrouillage (28, 30) sur l'axe de pivotement (24, 26).

3. Support de cartouche selon la revendication 2, dans lequel les éléments de verrouillage (28, 32 ; 30, 34) sont en liaison active par le biais d'un guidage à coulisse (48, 52 ; 50, 54).

4. Support de cartouche selon l'une des revendications 1 à 3, dans lequel une position de rinçage, une position d'insertion et une position de fonctionnement sont prévues comme positions de verrouillage.

5. Support de cartouche selon l'une des revendications précédentes, dans lequel le dispositif de verrouillage (20, 22) est agencé dans un espace intérieur (18) du bras de support (4, 6).

6. Support de cartouche selon l'une des revendications 2 à 5, dans lequel l'un des éléments de verrouillage (32, 34) du dispositif de verrouillage (20, 22) est soumis à une force d'enclenchement au moyen de laquelle l'élément de verrouillage (32, 34) est maintenu en prise avec sa voie de coulisse (52, 54) contre une voie de coulisse (48, 50) de l'autre élément de verrouillage (28, 30).

7. Support de cartouche selon la revendication 6, dans lequel les voies de coulisse (48, 52 ; 50, 54) du guide de coulisse sont réalisées de telle sorte que lors de l'application sur le bras de support (60.4) d'un couple de rotation autour de son axe de pivotement (26, 24), l'élément de verrouillage (32, 34) soumis à la force d'enclenchement, à partir d'une intensité déterminée du couple de rotation, est déplacé contre une direction d'exercice de la force d'enclenchement de façon à modifier les positions de verrouillage.

8. Support de cartouche selon la revendication 6 ou 7, dans lequel les voies de coulisse (48, 52 ; 50, 54) du guide de coulisse sont réalisées de telle sorte que sous l'effet de la force d'enclenchement au moins vers une position de verrouillage, le bras de support (6, 4) pivote en retour de façon autonome jusqu'à une déviation déterminée depuis cette position de verrouillage.

9. Support de cartouche selon l'une des revendications 6 à 8, dans lequel la force d'enclenchement est exercée sur l'élément de verrouillage (32, 34 ; 136) au moyen d'un ressort (40, 42 ; 140) prenant appui sur le bras de support (6, 4) ou sur l'axe de pivotement (26, 24).

10. Support de cartouche selon la revendication 9, dans lequel l'un des éléments de verrouillage (32, 34) est guidé en glissement dans un guidage du bras de support (6, 4) et soumis à la force d'enclenchement par le biais du ressort (40, 42) et dans lequel l'autre élément de verrouillage (28, 30) est réalisé sur une section d'axe (24, 26) de l'axe de pivotement (24, 26) agencée dans l'espace intérieur (18) du bras de support (6, 4).

11. Support de cartouche selon la revendication 10, dans lequel la voie de coulisse (48, 50) de l'élément de verrouillage (28, 30) réalisé sur la section d'axe s'étend dans la direction périphérique autour de l'axe de pivotement (26, 24) et la voie de coulisse (52, 54) de l'élément de verrouillage (32, 34) guidé en glissement dans le bras de support (6, 4) est réalisée sur une section d'extrémité de l'élément de verrouillage (32, 34) dirigée vers la section d'axe.

12. Support de cartouche selon la revendication 10, dans lequel la voie de coulisse (62) de l'élément de verrouillage (60) réalisé sur la section d'axe s'étend du côté frontal de l'axe de pivotement (26) et la voie de coulisse (66) de l'élément de verrouillage (64) guidé en glissement dans le bras de support (6) est réalisée sur un côté frontal de l'élément de verrouillage (64) dirigé vers la section d'axe.

13. Support de cartouche selon la revendication 9, dans lequel l'un des éléments de verrouillage (148) du dispositif de verrouillage est monté de façon fixe dans le bras de support (6), tandis que l'autre élément de verrouillage (136) est guidé en glissement et de façon non rotative sur l'axe de pivotement (26) et il est soumis par le biais du ressort (140) à la force d'enclenchement dans la direction de l'élément de verrouillage (148) monté dans le bras de support (6).

14. Support de cartouche selon la revendication 13, dans lequel l'élément de verrouillage (136) guidé sur l'axe de pivotement (36) est réalisé dans une forme annulaire et entoure l'axe de pivotement (26).

15. Support de cartouche selon la revendication 1, dans lequel :
- au moins un axe de pivotement (24, 26) est réalisé avec un certain nombre d'épaulements et/ou de renfoncements écartés les uns des autres dans la direction de pivotement, qui définissent les positions de verrouillage, et
- le dispositif de verrouillage (20, 22) possède au moins un piston (34) monté de façon à pouvoir coulisser axialement dans le bras de support correspondant, qui est réalisé sur son côté frontal avec une section de verrouillage compatible avec les épaulements et/ou les renfoncements et précontraint par ressort contre l'axe de pivotement (24, 26) du côté frontal.

16. Machine de dialyse comprenant un support de cartouche (1) selon l'une des revendications précédentes.
